# EUROPEAN PATENT APPLICATION

(11) **EP 2 353 402 A1**
(43) Date of publication of application: **10.08.2011**
(21) Application number: 10152841.2
(22) Date of filing: 05.02.2010
(51) Int. Cl.: A23L 1/22, A23L 1/025, A23L 1/33, A22C 29/04

(54) **Method for altering the flavor of a food product**

(71) Applicant: Sense for taste, 8310 Brugge (BE)
(72) Inventor: Lahousse, Bernard, 8500, Kortrijk (BE)
(74) Representative: Luys, Marie-José A.H.

(57) **Abstract**

A method for altering the flavor of a food product by extracting into a food product at least one substance derived from a solid compound, a liquid or a gas whereby the food product is exposed to a high pressure for a predetermined period of time together with the solid compound, the liquid or the gas, and in close proximity thereof.

## Description

The present invention relates to a method for altering the flavor of a food product by extracting into a food product at least one substance derived from a solid compound, a liquid or a gas.

The present invention also relates to a food product with an altered flavor whereby a substance derived from a solid compound, a liquid or a gas was extracted into the food product according to above mentioned method.

Impregnating or marinating of food in wine, spices, etc. at room temperature or below has been known for centuries. One huge disadvantage of this classical method of impregnation is the fact that this process is very slow, and thus very time-consuming. For example, in order to sufficiently impregnate meat with a specific flavor, it must be marinated for 2 to 3 days. Otherwise, the flavor isn't sufficiently absorbed into the meat.

Other examples of classical impregnation of food products are the pickling of meat or fish or the candying of fruits. Similar to marinating, these methods are also very slow and time-consuming.

More recently, impregnation of tastes in food products such as meat, fish, has been carried out by vacuum techniques. Hereby, a food product is placed in a vacuum for a certain period of time, together with a liquid. For example, an apple placed in an amount of red beetroot juice under vacuum pressure, will result in a red apple that is completely impregnated with the flavor of the red beetroot. Several examples of this technique are described by Frito et al, 1994 (Vacuum osmotic dehydratation, Process optimization and minimal processing of foods).

A vacuum causes the gas that is present in the food product to flow out. Simultaneously, new pores are created, which will be filled by the liquid in which the food product is immersed, as soon as the vacuum state is removed.

However, this technique can not be applied on all food products, and its efficiency is variable and very much depending of the specific application in which it is used.

On the other hand, the use of ultra high pressure (ex. 6000 bar), has up until now mainly been restricted to use as a cold alternative for the traditional pasteurization process, for example to kill off Salmonella, Vibrio and other pathogens. In these processes the main goal is to achieve a prolongation of the storage life.

A few exceptions to this rule are given below.

For example, ultra high pressure is used as an aid to open oysters and other shell-fish, without having to heat them or using mechanical tools to open them such as a knife.

Other advantages of ultra high pressure more specifically related to oysters are the following:
- the acceptation of oysters is higher. (Johnston, D. E., Farmer, L. J., Dynes, C., & Rutherford, J. A. (2003). High pressure processing of mussels, oysters and prawns. Conference, Pressure to Succeed-an Insight into High Pressure Food Processing, Ireland, 9 April 2003)
- oysters will automatically open as result of high pressure and the volume of the oyster will increase. (Lo´pez-Caballero, M. E., Pe'rez-Mateos, M., Montero, P., & Bonderi´as, A. J.(2000b). Oyster preservation by high-pressure treatment. Journal of Food Protection, 63, 196-201.Mackey, B. M., Forestie're, K., Isaacs).

Ultra high pressure has however never been described in the context of providing food products with specific tastes, flavors or other substances.

In the process of pickling meat however, normal pressure has already been applied. For example, brine is often injected in meat through needles, such as is shown in US 5934187, or if applied on a larger more industrial in WO2008135610.

A disadvantage of these techniques however, is the non-equal spreading of the fluid containing a certain aroma or flavor. Another advantage is the fact that this technique can not be applied on shell-fish since the loss of fluid during storage increases massively. A method in which brine is pressed into meat without needles are also known from US200800456.

Another example in which pressure is used to impregnate food is described in US2007059411. In US2007059411 a vacuum step is combined with a pressure step. The pressure is however restricted to 10 until 50000 Pa. The main goal of US2007059411 is to impregnate eggs with vitamin C.

Examples of the use of pressure in extraction applications are given below.

A combination of high pressure and extraction has been applied in patent CN 101323649 in order to extract polysaccharides out of shii-take mushrooms.

J6030703 describes the use of ultra high pressure of 500 to 12000 atm. to extract coffee and tea.

These different methods may be suitable for their specific applications, but will be insufficient as a generally applicable technique to impregnate food products with certain substances such as flavor or taste molecules.

There is thus a need for a more efficient method for altering the flavor of food products by extracting into a food product at least one substance derived from a solid compound, a liquid or a gas.

It is therefore a goal of the present invention to provide a method for altering the flavor of food products with a higher efficiency than the methods described in the prior art.

This goal is achieved with a method according to the characterizing part of the first claim.

Thereto, the food product is exposed to a high pressure for a predetermined period of time together with the solid compound, the liquid or the gas, and in close proximity thereof.

The inventors have surprisingly found that ultra high pressure can also be applied to impregnate food products with a certain flavor in a very short time span.

With this invention, food products can be impregnated under high pressure with for example aroma molecules, tastes, additives of a liquid, solid compound or a gas.

The advantage of this technique is that by only using natural products, for example ginger, basil, etc. a food product can completely absorb the taste of these products in a very short time.

A further advantage of the present invention is that products with a low storage life can also be impregnated given that the time to impregnate them is reduced from a couple of days to a couple of minutes such as for example is the case with shell-fish, more preferably mollusks, most preferably bivalves, such as oysters.

Preferably, the food product is chosen from the group consisting of shell-fish, fish, meat, vegetables, fruit, nuts, cereals, eggs, fungi, mushrooms, coffee, dairy, plants, herbs, spices, seaweeds, seeds, flowers and derivates thereof and combinations thereof.

The invention is however not restricted to this group, and all other food products known by the person skilled in the art, can also be used.

In a preferred embodiment of the present invention, the food product is chosen from the group of shell-fish, more preferably mollusks, most preferably bivalves.

Bivalves such as oysters and mussels have a shell consisting of two rounded plates called valves joined at one edge by a flexible ligament called the hinge.

Because the meat in bivalves is completely shut off from the outside world, it is very difficult to impregnate them with flavors or other substances. Often, the only way to transfer some taste to a bivalve is to open them, marinate them or the process them warm with a sauce.

With the method according to the present invention, food products contained in a shell such as bivalves can absorb tastes and other substances through their shell, which is a huge advantage.

Preferably, the solid compound, the liquid or the gas are chosen from the group consisting of food products, vegetables, fruit, meat, fish, shell fish, crustacean, nuts, cheese, cereals, spices, herbs, plants, coloring agents, softening agents, enzymes, gelling agents, wood, flowers, water, bouillon, fumet, vegetable juices, fruit juices, alcoholic drinks, seawater, sugar syrup, oil, molten chocolate, water extracts from cheese, condiments, fond, alcoholic drinks, spirits, liquors, beer, wine, soft drinks, energy drinks, dairy products, CO₂, helium, N₂, O₂, or a combination of two or more thereof.

The invention is however not restricted thereto and all other solid compounds, liquids or gases known by the person skilled in the art can also be used.

The substance is preferably chosen from the group of aromas, aromatic molecules, colorings agents, food additives, taste molecules, health improving components, texture changing components, vitamins, minerals and combinations of two or more thereof.

The invention is however not restricted thereto and all other solid substances known by the person skilled in the art can also be used.

The high pressure used in the present invention is preferably between 1000 - 6000 bar, more preferably between 2000 - 6000 bar, more preferably between 3000 - 6000 bar, most preferably between 4000 - 6000 bar.

In these pressure ranges, substances will optimally be transferred to the food product.

Preferably, the food product, together with the solid compound, the liquid or the gas, is exposed to a high pressure for a period of at least one second, preferably at least one minute, more preferably at least 3 minutes, but preferably no more than 5 minutes.

For very sensible food products, a time period of 1 second can be applied in order not to damage the food product. Generally speaking, 1 minute will be sufficient for a food product to absorb a substantial part of the substances from the solid compound, liquid or gas. Three minutes will be even better. Starting from 5 minutes, the amount of substances absorbed by the food product reduces drastically, and no additional benefits are gained from applying the method longer.

If the solid compound, the liquid or the gas, is a liquid, the food product is preferably in direct contact with the liquid.

The food product can for example be drowned in the liquid. This way, the food product will optimally absorb substances from the liquid.

In a preferred embodiment of the present invention, the food product is packed together with the solid compound, the liquid or the gas, in a packaging.

In a packaging the different ingredients will be very close to each other and substances will be optimally transferred.

Preferably, the food product is packed together with the solid compound, the liquid or the gas, in a vacuum packaging.

If the ingredients are packed in a vacuum packaging, the substances will be transferred even better.

In a preferred embodiment of the present invention, the food product is a liquid.

As such, liquids or fluids can be provided with different aromas, by treating these liquids such as for example water, wine, fruit and vegetable juices, etc. with a liquid, solid compound or gas under pressure.

The present invention further relates to a food product with an altered flavor whereby a substance derived from a solid compound, a liquid or a gas was extracted into the food product according to above mentioned method.

The present invention is further elucidated in the following description and examples, which illustrates preferred embodiments of the invention.

As mentioned above the invention can be applied on all sorts of food products. In a preferred embodiment of the invention however, shellfish are used. This can be crustaceans, mollusks, bivalves, lobster, cockles, venus shells, scallops, etc. In a more preferred embodiment bivalves such as mussels or oysters are used.

One specifically preferred embodiment is focused on oysters. The present market for fresh oysters is restricted to the natural fresh oyster. The only way to transfer some taste to the oyster is to open these, after which to marinate them of the process them warm with a sauce.

The use of ultra high pressure enables the facilitated opening of the oyster on the one hand while at the same time reducing the amount of Vibrio pathogens substantially, and on the other hand adjust the texture with respect to bite and volume.

This technique has already been applied by companies such as Gold Band Oysters in the USA and is promoted by producers of high pressure techniques such as Avure.

The inventors have however found that the use of ultra high pressure can also be applied on all sorts of food products in order to impregnate them with all kinds of aroma molecules, tastes, etc. in a very short time. Furthermore, also shell-fish, such as oysters can be impregnated with a taste or a flavor, whereas this was previously impossible since they are locked inside a shell. With this technique, it is for example possible to transfer the taste of ginger to an oyster by simple placing a piece of ginger near the shell of a fresh closed oyster. By means of ultra high pressure, the taste of the ginger will completely be absorbed in the oyster without the oyster losing its natural flavor. This technique is however not restricted to shell-fish, but can also be applied on fish, meat, vegetables, fruit, and all other food products known by the person skilled in the art.

Furthermore, the inventors have found that through high pressure, aromas can be extracted from solid or liquid products. For example, through combination of ginger and water, a watery extract of ginger can be obtained without the use of heat.

Different experiments have been conducted on a high pressure device from NC Hyperbaric with a 55 liter content per batch, at a temperature of 5-30°C, with a maximum pressure of 600 MPa (= 6000 bar).

The solid compounds, liquids, gases preferably contain aroma and taste molecules, but these can also be extended.

The solid compounds comprise food products such as vegetables, fruit, meat, fish, shell fish, crustacean, nuts, cheese, cereals, spices, herbs, plants, coloring agents, softening agents, enzymes, gelating agents or other additives. Also non-food products such as wood, flowers, etc. are comprised herein. From these solid compounds aromas, tastes, health improving components, texture changing components will be impregnated into a selected food product.

Liquids that are used can for example be water, bouillon, fumet, vegetable juices, fruit juices, alcoholic drink, seawater, sugar syrop, oil, molten chocolate, water extracts from cheese, et..

Gasses can for example be CO2, helium, N2, O2, ...

Below, some examples of specific applications of the present invention, are given.

### Example 1:

A fresh oyster was packed vacuum together with a piece of kiwi. The oyster was kept at 6000 bar for 1 minute.

Result: the oyster has a soft taste of kiwi combined with the natural taste of a fresh oyster.

The texture has slightly evolved because of the high pressure so that an oyster was obtained with a little more bite, which was positively received by the taste panel. (The latter was already known by Johnston et al, 2003)

### Example 2:

A fresh oyster was packed in a vacuum together with champagne. The oyster was kept at 6000 bar for 5 minutes.

Result: the oyster has the taste of the champagne. Furthermore, also the CO2 is absorbed in the oyster, as a result of which a sparkling sensation is experienced upon eating the oyster.

### Example 3:

A piece of lamb meat of 100 g was packed together with 5 grams of seaweed and 20 grams of bouillon. The meat was kept at 6000 bar for one minute.

Result: the meat is fully impregnated with the taste of the seaweed.

### Example 4:

An eggplant was packed in a vacuum together with bouillon. The eggplant was kept at 6000 bar for five minutes.

Result: the eggplant was completely filled with a liquid tasting of eggplant and bouillon. The liquid could be drunk from a hole in the eggplant.

### Example 5:

Fish was packed together with olive oil. The fish was kept at 6000 bar for 1 minute, after which it was unpacked and rinsed.

Result: The taste of olive oil was present in the fish without the fish feeling greasy.

### Example 6:

Water was packed together with seaweed and a piece of lamb meat. This mixture was treated with a pressure of 6000 bar for five minutes.

Result: the water tasted like lamb and seaweed. This liquid could be used as a basis for sauces.

### Example 7:

Wine was packed together with lemongrass. The mixture was treated with a pressure of 6000 bar for five minutes.

Result: the wine had the aromas of lemongrass.

### Example 8:

Olive oil was packed together with lemongrass. The mixture was treated with a pressure of 6000 bar for five minutes.

Result: The olive oil had the taste of lemongrass.

## Claims

1. A method for altering the flavor of a food product by extracting into a food product at least one substance derived from a solid compound, a liquid or a gas **characterized in that** the food product is exposed to a high pressure for a predetermined period of time together with the solid compound, the liquid or the gas, and in close proximity thereof.

2. A method according to claim 1 **characterized in that** the food product is chosen from the group consisting of shell-fish, fish, meat, vegetables, fruit, nuts, cereals, eggs, fungi, mushrooms, coffee, dairy, plants, herbs, spices, seaweeds, seeds, flowers and derivates thereof and combinations thereof.

3. A method according to claim 1 or 2 **characterized in that** the food product is chosen from the group of shell-fish, more preferably mollusks, most preferably bivalves.

4. A method according to any one of claims 1-3, **characterized in that** the solid compound, the liquid or the gas are chosen from the group consisting of food products, vegetables, fruit, meat, fish, shell fish, crustacean, nuts, cheese, cereals, spices, herbs, plants, coloring agents, softening agents, enzymes, gelling agents, wood, flowers, water, bouillon, fumet, vegetable juices, fruit juices, alcoholic drinks, seawater, sugar syrup, oil, molten chocolate, water extracts from cheese, condiments, fond, alcoholic drinks, spirits, liquors, beer, wine, soft drinks, energy drinks, dairy products, CO₂, helium, N₂, O₂, or a combination of two or more thereof.

5. A method according to any one of claims 1-4, **characterized in that** the substance is chosen from the group of aromas, aromatic molecules, colorings agents, food additives, taste molecules, health improving components, texture changing components, vitamins, minerals and combinations of two or more thereof.

6. A method according to any one of claims 1-5, **characterized in that** the high pressure is preferably between 1000 - 6000 bar, more preferably between 2000 - 6000 bar, more preferably between 3000 - 6000 bar, most preferably between 4000 - 6000 bar.

7. A method according to any one of claims 1-6, **characterized in that** the food product, together with the solid compound, the liquid or the gas, is exposed to a high pressure for a period of at least one second, preferably at least one minute, more preferably at least 3 minutes, but preferably no more than 5 minutes.

8. A method according to any one of claims 1-7, **characterized in that** the solid compound, the liquid or the gas, is a liquid whereby the food product is in direct contact with the liquid.

9. A method according to any one of claims 1-8, **characterized in that** the food product is packed together with the solid compound, the liquid or the gas, in a packaging.

10. A method according to any one of claims 1-9, **characterized in that** the food product is packed together with the solid compound, the liquid or the gas, in a vacuum packaging.

11. A method according to any one of claims 1-10, **characterized in that** the food product is a liquid.

12. A food product with an altered flavor whereby a substance derived from a solid compound, a liquid or a gas was extracted into the food product according to the method of any one of claims 1-11.
